# EUROPÄISCHE PATENTSCHRIFT

(11) **EP 0 882 456 B1**
(45) Veröffentlichungstag und Bekanntmachung des Hinweises auf die Patenterteilung: **12.03.2003**
(21) Anmeldenummer: 98110126.4
(22) Anmeldetag: 04.06.1998
(51) Int. Cl.: A61L 2/26, G01N 31/22, C12Q 1/22, A61L 2/28

(54) **Sterilisationstestvorrichtung**
Sterilisation test device
Dispositif de test pour la stérilisation

(30) Priorität: 07.06.1997 DE 19724155
(43) Veröffentlichungstag der Anmeldung: 09.12.1998
(73) Patentinhaber: SECUNDUS Medizinische Kontrollsysteme GmbH, 41516 Grevenbroich (DE)
(72) Erfinder: Kewitsch, Günter, 41516 Grevenbroich (DE); Schefter, Siegfried, 41516 Grevenbroich (DE)
(74) Vertreter: von Creytz, Dietrich, Dipl.-Phys.

(56) Entgegenhaltungen:
- WO-A-98/25120
- DE-A- 4 218 890
- US-A- 4 272 480

## Beschreibung

Die Erfindung betrifft eine Sterilisationstestvorrichtung mit einer in einem Prüfkörper integrierten und zur Aufnahme eines Indikators vorgesehenen Testkammer, die vorgegebene Zuleitungsmittel für das jeweilige Sterilisationsmittel sowie eine mit einem Kammerdeckel zu verschließende Kontrollöffnung besitzt und außer am Einlaß der Zuleitungsmittel mit einer Testkammerdichtung gegen ein Eindringen des Sterilisationsmittels ausgestattet ist. Sie betrifft ferner ein Verfahren zum Betrieb der Vorrichtung.

Die Testvorrichtung umfaßt einen Prüfkörper. Derartige Testvorrichtungen bzw. Prüfkörper werden mit langen Zuleitungen ausgestattet. Sie sollen es erlauben, bei dem jeweiligen Sterilisationsverfahren mit ausreichender Wahrscheinlichkeit anzuzeigen, ob die jeweilige Sterilisation erfolgreich war oder nicht. Als Sterilisationsmittel wird bevorzugt eine gas- oder dampfförmige Sterilisationsatmosphäre, z.B. mit Wasserdampf, Ethylenoxid oder Formaldehyd, vorgesehen.

In DE 87 00 471 U wird ein Prüfkörper beschrieben, der in seiner Testkammer einen Chemo-Indikator aufnimmt und der insgesamt in einen Sterilisationsraum zu setzen ist. Die Zuleitungsmittel des Prüfkörpers werden so ausgebildet, daß sie schwer zu sterilisierende Bedingungen simulieren. Zu diesem Zweck wird als Zuleitungsmittel ein relativ langer Schlauch mit gegenüber der Testkammer relativ kleinem Durchmesser vorgesehen, der den einzigen Zugang des Sterilisationsmittels zur Testkammer bilden soll.

Speziell bei der sogenannten Dampfsterilisation, z.B. im Krankenhausbereich, ist die lange enge Zuleitung der Testkammer sehr vorteilhaft. Bei diesem Verfahren wird nämlich der Sterilisator vor dem Einlassen des z.B. Üblicherweise 121 oder 134 °C heißen Sattdampfes evakuiert. Dann kann die im Sterilisationsraum ursprünglich befindliche Luft den Zugang des Wasserdampfes nicht mehr behindern. Da eine lange dünne Leitung nur schwer vollständig zu entlüften ist, zeigt ein in der Testkammer (also am geschlossenen Ende der Leitung) angeordnete, vorschriftsmäßig veränderter Indikator auch an, daß der Sterilisationsraum vor dem Durchdämpfen ausreichend entlüftet war.

Eine Testvorrichtung eingangs genannter Art wird auch in EP 0 628 814 A1 beschrieben. Auch diese Testvorrichtung wird im Krankenhausbereich zusammen mit oder auch ohne Sterilisationsgut in einen Sterilisator gegeben, wobei das Sterilisationsmittel durch die gesamte, als dünner Schlauch ausgebildete Zuleitung, strömen soll, um einen in der Testkammer angeordneten Indikator zu erreichen. Ein Farbumschlag (oder dergleichen Änderung) des Indikators zeigt an, ob ausreichende Sterilisationsbedingungen geherrscht haben oder nicht. Auf diese Weise kann indirekt auf eine korrekte Sterilisation des Sterilisationsgutes geschlossen werden.

Gemäß vorgenannter EP 0 628 814 A1 wird bei der Dampfsterilisation der Sterilisator bzw. Sterilisationsraum vor dem Einführen von Sattdampf evakuiert. Anschließend wird der Dampf für eine Zeitdauer von z.B. 4 bis 6 Minuten bei einer Temperatur von 134 °C oder beispielsweise für 15 bis 30 Minuten mit einer Temperatur von etwa 121 °C in den Sterilisationsraum geleitet.

Unter den vorgenannten Bedingungen sind nach DE 43 19 397 C1 Beschädigungen der Zuleitung denkbar. Die Beschädigung kann mit einer mit wirtschaftlichen Mitteln kaum nachprüfbaren Mikroporosität, beispielsweise an der Verbindung bzw. Kupplung zwischen Zuleitung und Testkammer, beginnen. Schon geringe Undichtheiten können ausreichen, den Weg der umgebenden Sterilisationsatmosphäre zum Indikator - unter Umgehung des langen Wegs durch die Zuleitung - abzukürzen, so daß der in der Testkammer befindliche Indikator fälschlicherweise eine vollständige Sterilisation bzw. ausreichende Sterilisationsbedingungen anzeigt.

In der bisherigen Praxis können Testkammer und Zuleitung aus gleichen oder verschiedenen Materialien, wie Metall, Gummi, Kunststoff usw., bestehen. Wichtig ist vor allem, daß die Materialien den thermischen und mechanischen Belastungen im Sterilisator dauerhaft standhalten und daß sie die zu sterilisierenden Gegenstände nicht beeinträchtigen. Die Verbindung der Zuleitung an der Testkammer bzw. deren Einlaß wird je nach Material geschweißt, gelötet, geschraubt, gesteckt, mit Manschetten gedichtet und zugleich gegen ein Abknicken gesichert sowie auf viele andere übliche Weisen gebildet.

Nach einer der Erfindung zugrundeliegenden Erkenntnis, bleibt diese Kuppelstelle jedoch immer ein Schwachpunkt des ganzes Testsystems, weil die Zuleitung lose an der Testkammer bzw. deren Behälter hängt und bei Anwendung des Prüfkörpers die Kuppelstelle fast immer mechanisch belastet wird. Irgendwann ist daher mit an Sicherheit grenzender Wahrscheinlichkeit mit einer Undichtheit an dieser Stelle zu rechnen, so daß die Atmosphäre des den Prüfkörper im Sterilisator umgebenden Sterilisationsmittels im Kurzschluß - unter Umgehung der langen Zuleitung - in die Testkammer gelangen kann. Selbst bei der ersten Anwendung kann die Kuppelstelle in unzulässiger Weise belastet und beschädigt werden. Daher wird nach der oben genannten DE 43 19 397 C1 die als Schlauch ausgebildete Zuleitung durch einen Pfropfen aus porösem Material ersetzt, der das Einströmen des Sterilisationsmittels in die Testkammer wie eine Labyrinthdichtung erschwert. Nach EP 0 657 177 A2 kann als poröses Material des Pfropfens auch hydrophiles Material eingesetzt werden.

Nach einer der Erfindung zugrundeliegenden Erkenntnis kann der Zugang des Sterilisationsmittels zum Innern der Testkammer und damit zum Indikator nicht nur an der Verbindungsstelle zwischen Zuleitung und Testkammer, sondern auch an der Dichtung einer der Testkammer zugeordneten Kontrollöffnung - gewissermaßen als Kurzschluß unzulässig verkürzt werden. Die Testkammerdichtung besteht also aus den Dichtmitteln an der Zuleitung bzw. deren Kupplung am Prüfkörper und aus den Dichtmitteln der Kontrollöffnung

Die Kontrollöffnung kann dazu dienen, einen in der Testkammer befindlichen Indikator in Augenschein zu nehmen zu prüfen bzw. den Indikator einzusetzen oder herauszunehmen. In der Praxis kann die Kontrollöffnung mit einem Deckel, beispielsweise mit Hilfe einer Schraubkappe, bevorzugt mit Dichtungsring, verschlossen werden (vgl. die oben genannten Druckschriften DE 87 00 471 U, EP 0 628 814 A1 und DE 43 19 397 C1). Der jeweilige Deckel bzw. dessen Dichtung können den Sterilisationsbedingungen voll ausgesetzt werden. Die Schraubkappe soll nach jedem Einsatz geöffnet werden, um den Indikator auszutauschen. Die thermische und/oder mechanische Belastung der Deckeldichtung kann daher zu Beschädigungen führen, mit dem Ergebnis, daß ein Leck mit einem Weg für das Sterilisationsmittel unmittelbar durch die Dichtung hindurch in die Testkammer entsteht. Eine Überprüfung dieser Dichtung ist mit ausreichender Sicherheit wirtschaftlich nicht möglich. Es bleibt daher keine Wahl, als den Dichtkörper häufig zu wechseln und zu hoffen, daß er zumindest bei der ersten Benutzung die an ihn gestellten Anforderungen erfüllt. Eine Sicherheit dafür gibt es jedoch nicht.

Der Erfindung liegt die Aufgabe zugrunde, ein Mittel zur sicheren Prüfung der Dichtung der Testkammer zu schaffen.

Die erfindungsgemäße Lösung besteht nach Anspruch 1 für die Sterilisationstestvorrichtung darin, daß in der Testkammerdichtung im Weg eines gegebenenfalls die Zuleitungsmittel verkürzend umgehenden Lecks mindestens ein an eine zur Aufnahme eines Hilfsindikators vorgesehene Hilfskammer angeschlossener Ableitkanal vorgesehen ist. Vorzugsweise soll sich erfindungsgemäß um die Testkammer im Weg eines die Zuleitungsmittel umgehenden Lecks der Testkammerdichtung mindestens ein an die luftdicht gekapselte Hilfskammer angeschlossener Ringkanal erstrecken. Einige Verbesserungen und weitere Ausgestaltungen der Erfindung werden in den übrigen Ansprüchen beschrieben.

Die erfindungsgemäße Prüfvorrichtung enthält in der Hilfskammer einen weiteren Indikator, den sogenannten Hilfsindikator, der bei einwandfrei funktionierender Sterilisationstestvorrichtung unverändert bleibt. Wenn dagegen der Hilfsindikator eine Berührung mit der Sterilisationsatmosphäre, z.B. durch Verfärbung, anzeigt, ist eine ordnungsgemäße Sterilisation nicht gesichert. Es kann dann nämlich sein, daß die Testkammer, z. B. an der Kontrollöffnung oder an der Verbindung von Prüfkörper und Zuleitung, nicht ordnungsgemäß abgedichtet war. Für ein Verfahren zum Betrieb der eingangs genannten Vorrichtung heißt das, daß außerhalb der Testkammer (aber im Bereich des Prüfkörpers) mit Hilfe des Hilfsindikators geprüft wird, ob die Testkammerdichtung einwandfrei wirkt, das heißt dichtet.

Grundsätzlich kann der Hilfsindikator ebenso wie der Hauptindikator ausgebildet werden und reagieren. Da aber der Hilfsindikator keine unmittelbare Aussage über den Sterilisationsprozeß an sich liefern soll, kann es günstig sein, ihn deutlich empfindlicher gegenüber Einwirkungen des Sterilisationsmittels zu machen.

In der erfindungsgemäßen Vorrichtung wird der Hilfsindikator durch das Sterilisationsmittel verändert, wenn die Testkammerdichtung irgendwo defekt ist. Zu diesem Zweck werden die Hilfskammer und deren Ableit- bzw. Ringkanäle so angeordnet bzw. mit solchen Stichkanälen ausgestattet, daß das den Raum um die Prüfvorrichtung füllende Sterilisationsmittel durch das jeweilige Leck zuerst in die sich bevorzugt außen an die Testkammer anschließende Hilfskammer fließen muß. Erfindungsgemäß wird also bei defekter Testkammerdichtung das durch ein Leck - gewissermaßen im Kurzschluß unter Umgehung der Zuleitungsmittel von außen in Richtung Testkammer fließende Sterilisationsmittel vor Erreichen der Testkammer bereits in (gegebenenfalls durch) die Hilfskammer geleitet. In einem solchen Fall wird der Hilfsindikator durch das in die Hilfskammer eingedrungene Sterilisationsmittel verfärbt oder in anderer Weise verändert. Ohne aufwendige Prüfung kann dann oft nicht entschieden werden, welche Dichtung der Testkammer undicht bzw. welcher Grund für die Veränderung des Hilfsindikators vorlag. Aus diesem Grunde soll die Sterilisation wiederholt werden, wenn der Hilfsindikator aus irgendeinem Grund verändert ist.

Nach Vorstehendem kann die Erfindung in im Prinzip gleicher Weise zur Prüfung der Testkammerdichtung sowohl an der Kontrollöffnung der Testkammer als auch an der Kupplung (Verbindung) von Prüfkörper und Zuleitungsmitteln eingesetzt werden. An diesen beiden kritischen Bereichen kommen im Rahmen der Erfindung verschiedene Lösungswege für verschieden ausgebildete Vorrichtungen in Frage.

Hierzu gehört eine erste Sterilisationstestvorrichtung mit (abgedichtet angeschlossenen) Zuleitungsmitteln für das Sterilisationsmittel und gegen ein Eindringen des letzteren mit Hilfe einer Kammerdichtung verschließbarer Kontrollöffnung. Für diese Vorrichtung besteht die erfindungsgemäße Lösung insbesondere darin, daß sich jenseits der Kammerdichtung außen an die Testkammer - das heißt funktionell außerhalb der vorgeschriebenen Testkammeratmosphäre - eine bei mangelhafter Kammerdichtung durch die Testkammer hindurch von dem Sterilisationsmittel geflutete Hilfskammer (mit Hilfsindikator) anschließen.

Bei dieser Ausbildung gemäß weiterer Erfindung werden letztlich an der Kontrollöffnung zwei (auf einem theoretischen Leckweg des Sterilisationsmittel) hintereinander geschaltete Dichtungen vorgesehen. Dadurch allein wird die Dichtung der Kontrollöffnung zwar verbessert, aber nicht gesichert. Die Zuverlässigkeit der beiden hintereinander geschalteten Dichtungen kann erst durch den in erfindungsgemäßer Weise wischen die beiden Dichtungen gesetzten Hilfsindikator geprüft und damit garantiert werden.

Gemäß weiterer Erfindung wird bei Verwendung einer die Kontrollöffnung mit Hilfe der Kammerdichtung verschließenden Kammerkappe, insbesondere Klemm- oder Schraubdeckel, über die Kammerkappe eine Überkappe, insbesondere als Klemm- bzw. Schraubdeckel, mit einer Außendichtung zum Bilden der Hilfskammer zwischen der Kammerdichtung und der Außen- bzw. Hilfsdichtung gestülpt. In dieser Ausführungsform soll also die Überkappe die Kammerkappe ganz übergreifen und jede der Kappen soll mit ihrer Dichtlippe einen Dichtring gegen eine zugehörige Dichtlippe der Testkammer pressen können. Außer der Testkammer mit dem Hauptindikator steht dann - zwischen den beiden Dichtungen - eine Hilfskammer mit dem Hilfsindikator zur Verfügung.

Wenn eine solche Konstruktion betreffend Herstellung und/oder Bedienung zu aufwendig sein sollte, kann beispielsweise gemäß noch weiterer Erfindung vorgesehen werden, als Kammer- und Hilfsdichtung einen einzigen Doppeldichtring zwischen den - in eine Ebene gelegten - Dichtlippen von Test- und Hilfskammer vorzusehen. Der Doppeldichtring soll gegebenenfalls einen (radial) inneren Dichtstreifen zum Abdichten der Testkammer und einen (radial) äußeren Dichtstreifen für die Dichtlippe zum Abdichten der Hilfskammer aufweisen. Als Grenze zwischen den Dichtstreifen soll auf Ober- und Unterseite des Doppeldichtrings je eine umlaufende Rinne - als axiale Ausnehmung des Ringkörpers - vorgesehen werden, wobei die Hilfskammer mit der von der Testkammer abgewandten Rinne in Verbindung stehen soll. Schließlich sollen die beiden Rinnen an wenigstens einer Stelle durch ein (axiales) Loch miteinander verbunden werden. Die Rinnen sind dabei als Leitungsweg für das Sterilisationsmittel gedacht und ausgebildet.

Wenn bei einer der beschriebenen Konstruktionen der Doppeldichtring an irgendeiner Stelle des die Testkammer abdichtenden Dichtstreifenteils zugewandten Seite eine Undichtheit in radialer Richtung aufweisen oder erhalten sollte, kommt das Innere der Testkammer über die Undichtheit, die der Testkammer zugewandte Rinne, das Loch und die gegenüberliegende Rinne mit der Hilfskammer in Verbindung. Im Ergebnis wirkt dann die Sterilisationsatmosphäre bis in die Hilfskammer, so daß der dortige Hilfsindikator verändert werden kann.

Noch einfacher wird die erfindungsgemäße Lösung, wenn gemäß weiterer Erfindung eine Hilfskammer mit einer Kontrollöffnung am Prüfkörper räumlich direkt neben der Testkammer angeordnet, ein die Testkammer-Kontrollöffnung umgebender, zur Hilfskammer führender Ableitkanal vorgesehen wird und dabei beide Kontrollöffnungen mit ein und derselben Dichtung eines Deckels verschließbar sind. Es kann zu diesem Zweck ein einfacher Schraubdeckel vorgesehen werden. Wenn die beiden Kontrollöffnungen in einer Ebene liegen, genügt innerhalb der Deckelkappe eine einzige im wesentlichen ebene Dichtplatte, insbesondere aus Silikon.

Wie gesagt, hat die Erfindung alternativ oder zugleich sowohl an der Dichtung der Kontrollöffnung als auch an dem Anschluß der Zuleitungsmittel Bedeutung. Für den letzteren Fall besteht die weitere Erfindung darin, daß als Schlauch ausgebildete Zuleitungsmittel vorgesehen werden und der Prüfkörper einen mit einem Längsdurchgang zur Testkammer ausgestatteten Stutzen (Schlauchstutzen) zum Aufschieben eines Schlauchendes ausgestattet wird. Auf dem Stutzenumfang soll mindestens ein (nach dem Aufschieben des Schlauchs von diesem eingekammerter) in Richtung des Stutzenumfangs geschlossener und mit der Hilfskammer verbundener Ableitkanal vorgesehen werden. Zur Kontrolle der Dichtheit an der Kuppelstelle zwischen Zuleitung und Prüfkörper kann also dieselbe Hilfskammer und demgemäß derselbe eine Hilfsindikator verwendet werden, der auch zur Prüfung der Testkammerdichtung an deren Kontrollöffnung vorgesehen wird. Auf den beschriebenen beiden "Seiten" der Testkammer läßt sich also das im Prinzip selbe Prüf- bzw. Meßprinzip einsetzen.

Vorzugsweise wird an der Verbindungsstelle von Zuleitungsmittel und Prüfkörper ein Stutzen mit einem sich in den jeweiligen Schlauch hinein erstreckenden Konusteil vorgesehen (der Konus verjüngt sich in Richtung auf das Schlauchinnere), wobei der Ableitkanal im Bereich des Konusteils auf dem Konusumfang vorgesehen wird. Die vorgenannte Verbindung zwischen Ableitkanal und Hilfskammer kann teilweise oder ganz innerhalb des Prüfkörpers verlaufen. Wenn es Probleme bereitet, die Verbindung bis tief in den Konus hinein (in Richtung auf dessen freies Ende) innerhalb des Prüfkörpers zu führen, kann die Verbindung auch als Rinne außen auf dem Konus entlang geführt werden. Die Rinne wird ja durch den übergestülpten Schlauch nach außen hin verschlossen. Irgendwann, noch innerhalb des Schlauchs, soll dann allerdings die Verbindung in das Innere des Prüfkörpers übergehen. An dieser Stelle kann zweckmäßig ein zweiter Ableitkanal, das heißt bevorzugt ein den Konus umgebender Ring- oder Umfangskanal, vorgesehen werden.

Bei Anwendung der Erfindung im Bereich der Schlauchkupplung kann es vorteilhaft sein, möglichst den gesamten Schlauchteil auf Dichtheit prüfen zu können, der über dem Konus gestülpt wird. Im Bereich des Konus gibt es zumindest zwei unterschiedliche Zonen: In einer ersten an die Konusspitze (das freie Ende) angrenzenden Zone dringt das Sterilisationsmittel - speziell so lange der Druck noch nicht ausgeglichen ist - vom Schlauchinnern her zwischen Schlauchinnenfläche und Konusaußenfläche ein. Diese erste Zone kann, wenn der Außendurchmesser des freien Konusendes nur wenig kleiner als die lichte Weite des Schlauchs (z.B. im Verhältnis 1 : 10 mm bei einer Konussteigung von 5 bis 10°) auf eine geringe Breite (in Schlauchlängsrichtung mit den vorgenannten Zahlen, z.B. 10 mm) beschränkt werden. Oberhalb (gesehen in Richtung der Konussteigung) der ersten Zone schließt sich eine zweite Zone an. Der gegebenenfalls auf dem Konusumfang vorgesehene Ableitkanal muß natürlich in der zweiten Zone positioniert werden, in die normalerweise vom Schlauchinnern her Sterilisationsmittel nicht vordringen kann. Unter dieser Prämisse soll der Ableitkanal aber innerhalb der zweiten Zone möglichst nahe an die genannte erste Zone gelegt werden.

Wenn der Ableitkanal in der zweiten Zone positioniert wird, kann bei unversehrtem bzw. einwandfreiem Schlauch kein Sterilisationsmittel in den Ableitkanal eindringen. Befindet sich aber irgendwo in dem Teil des Schlauchs, der über den Konus gestülpt ist, eine Undichtheit oder eine unzulässige Unebenheit, kann eventuell Sterilisationsmittel durch die Fehlstelle zu dem Ableitkanal und damit zur Hilfskammer gelangen. Wichtig ist dabei, daß das durch die Fehlstelle des Schlauchs eindringende Sterilisationsmittel zuerst und zumindest auch in den Ableitkanal (und keinesfalls nur in den Konuseingang der Testkammer) fließt.

Wenn die Undichtheit des Schlauchs in dem Bereich in der oben definierten ersten Zone in der Nachbarschaft des freien Konusendes auftritt, ist der Abstand der jeweiligen Fehlstelle zum Ableitkanal auf jeden Fall kleiner als der größere Weg bzw. Abstand des Ableitkanals zum freien Konusende. Da aber das Sterilisationsmittel - so lange ein Unterschied zwischen dem Innen- und Außendruck herrscht - in der Lage ist, den größeren der beiden Wege zwischen Schlauch und Konusoberfläche zurückzulegen, führt ein, auch ein in der ersten Zone vorhandener Fehler, z.B. ein Loch im Schlauch, - also ein Fehler - zwischen freiem Konusende und Ableitkanal - dazu, daß Sterilisationsmittel bis zum Ableitkanal und damit bis zur Hilfskammer und deren Indikator vordringen kann. Auch für die erste Zone (und natürlich die zweite Zone) läßt sich also durch die Erfindung ausschließen, daß etwa ein oben definierter Kurzschluß das Meßergebnis in der Testkammer unbemerkt verfälscht.

Bei Betrieb einer Sterilisationstestvorrichtung der vorliegenden Art können Probleme auftreten, wenn aus irgendwelchen Gründen, z.B. durch Unachtsamkeit, nach dem Erzeugen des Vakuums im Sterilisator nicht Sattdampf, sondern ungesättigter bzw. überhitzter Dampf angelangt. Wenn der in der Testkammer befindliche Indikator nicht zwischen Sattdampf und leicht überhitztem Dampf unterscheidet, können Fehlinterpretationen betreffend das Sterilisationsergebnis auftreten.

Gemäß weiterer Erfindung wird daher das freie Ende eines jeweiligen Zuleitungsschlauchs oder dergleichen - alternativ ein Teil der Testkammer bzw. einer erweiterten Testkammer - mit einem von dem Sterilisationsmittel auf dem Weg zum Hauptindikator unbedingt zu durchdringenden Pfropfen aus hydrophilem Material gefüllt. Wenn unter diesen Umständen ungesättigter Dampf in den Sterilisator gelangt, wird der Dampf durch den Pfropfen aus hydrophilem Material noch weiter getrocknet, so daß er im Bereich des Indikators praktisch trocken ist und diesen nicht mehr verändern kann.

Anhand der schematischen Darstellung von Ausführungsbeispielen werden einige Einzelheiten der Erfindung erläutert. Es zeigen:
- **Fig. 1**: einen Längsschnitt durch ein erstes Ausführungsbeispiel einer erfindungsgemäßen Sterilisationstestvorrichtung;
- **Fig. 2**: ein zweites Ausführungsbeispiel der erfindungsgemäßen Sterilisationstestvorrichtung;
- **Fig. 3 und 4**: eine vergrößerte Darstellung im Längs- und Querschnitt von Teilen der Sterilisationstestvorrichtung nach Fig. 2; und
- **Fig. 5 und 6**: ein bevorzugtes Ausführungsbeispiel der erfindungsgemäßen Sterilisationstestvorrichtung im Längs- und Querschnitt.

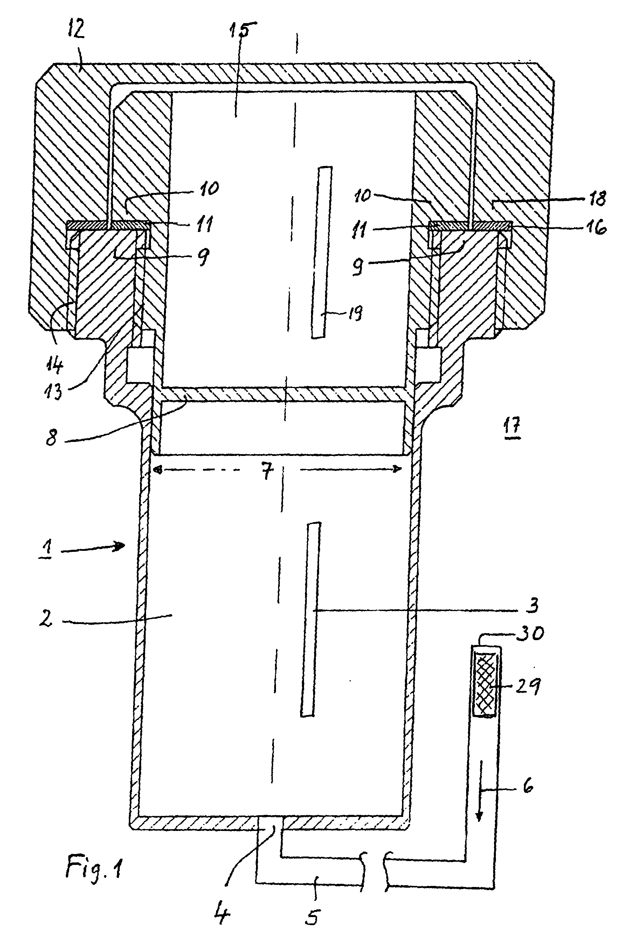

Die insgesamt mit 1 bezeichnete Sterilisationstestvorrichtung nach Fig. 1 enthält in einer Testkammer 2 einen Hauptindikator 3. Die Testkammer besitzt einen Anschluß 4 zum Einlaß des jeweiligen Sterilisationsmittels. An den Anschluß 4 werden außen Zuleitungsmittel 5 angekuppelt, die einen derartig langen bzw. (auch innen) als Labyrinth ausgebildeten Weg für das in Pfeilrichtung angedeutete Sterilisationsmittel 6 darstellen, daß angenommen werden kann, das Sterilisationsmittel an jeden Punkt des umgebenden Sterilisationsraums, in der die Vorrichtung 1 bei Betrieb liegt, gelangt, bevor es in der Testkammer 2 ankommt.

Die Testkammer 2 besitzt nach Fig. 1 eine, z.B. beim Wechsel des Hauptindikators 3 zu benutzende, Kontrollöffnung 7, einen auf die Kontrollöffnung 7 zu setzenden Deckel 8 und eine zwischen Dichtlippe 9 der Testkammer 2 und eine Dichtlippe 10 des Deckels 8 zu legende Kammerdichtung 11. Die Kammerdichtung 11 soll so ausgebildet sein, daß sie einen unmittelbaren Durchtritt des die Vorrichtung 1 gegebenenfalls umgebenden Sterilisationsmittels 6 ausschließt.

Im Ausführungsbeispiel nach Fig. 1 wird außen über den Deckel 8 ein Hilfsdeckel 12 gestülpt, der ebenso wie der Deckel 8 als Schraubdeckel mit Gewinde 13 bzw. 14 ausgebildet werden kann und zwischen sich und dem Deckel 8 eine Hilfskammer 15 einschließt. Die Hilfskammer 15 wird mit der Kammerdichtung 11 gegenüber der Testkammer 2 und mit einer Hilfsdichtung 16 gegenüber dem Außenraum 17 abgedichtet, so daß die Hilfskammer 15 bei Betrieb und einwandfreien Dichtungen einen völlig luftdicht abgeschlossenen Raum bildet. Die Hilfsdichtung 16 wird im Ausführungsbeispiel zwischen der Dichtlippe 9 der Testkammer 2 und einer Dichtlippe 18 des Hilfsdeckels 12 geklemmt.

Bei Anwendung wird die Vorrichtung 1 mit einem Hauptindikator 3 in der Testkammer 2 und einem Hilfsindikator 19 in der luftdicht verschlossenen Hilfskammer 15 irgendwo zwischen die zu sterilisierenden Sachen in den jeweiligen Sterilisationsraum gesetzt. Nach Abschluß der Sterilisation wird der Hilfsdeckel 12 abgeschraubt, wenn der Hilfsindikator 19 unverändert ist, hat die Sterilisationstestvorrichtung 1 mit größter Wahrscheinlichkeit einwandfrei gearbeitet (über das Ergebnis des Sterilisationsprozesses an sich ist damit noch nichts gesagt). Ist dagegen der Hilfsindikator 19 irgendwie verändert, kann das bedeuten, daß die Sterilisationstestvorrichtung 1 nicht ordnungsgemäß gearbeitet hat. Es wird auch hierdurch keine Aussage über das Ergebnis der Sterilisation an sich gemacht. Trotzdem muß die Sterilisation wiederholt werden, weil nicht mehr sicher festzustellen ist, wodurch eine Veränderung des Hauptindikators 3 begründet war.

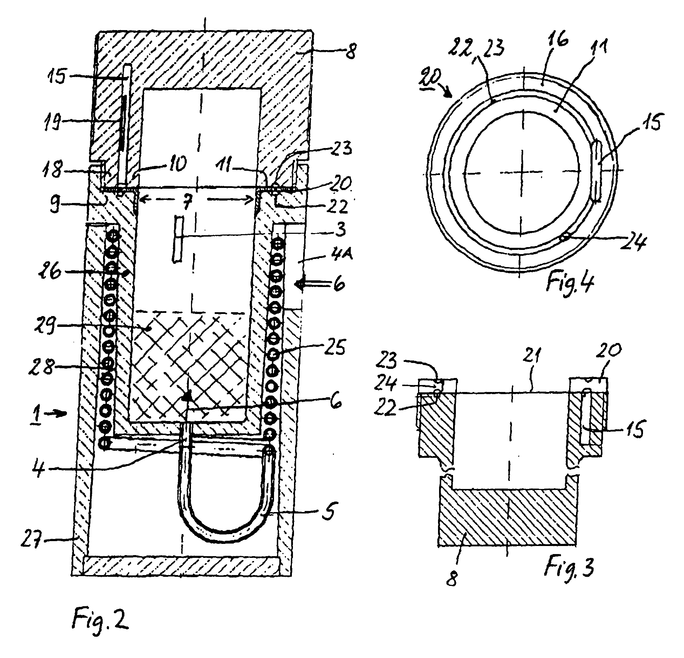

Fig. 2 zeigt ein weiteres Ausführungsbeispiel einer insgesamt mit 1 bezeichneten erfindungsgemäßen Sterilisationstestvorrichtung. In diesem Fall wird ein einziger die Kontrollöffnung 7 mit Hilfe einer Kammerdichtung 11 verschließender Kammerdeckel 8, insbesondere Schraubdeckel, vorgesehen. Die Testkammer 2 und die Hilfskammer 15 besitzen wiederum Dichtlippen 9, 10 und 18.

Wie auch in Fig. 3 und 4 dargestellt, wird nach Fig. 2 als Kammer- und Außendichtung ein einziger Doppeldichtring 20 zwischen den - in etwa in einer Ebene 21 liegenden - Dichtlippen 9 bzw. 10 und 18 von Testkammer 2 und Hilfskammer 15 vorgesehen. Der Doppeldichtring 20 besitzt einen (radial) inneren Dichtstreifen 11 für die Dichtlippenpaarung 9, 10 der Testkammer 2 und einen (radial) äußeren Dichtstreifen 16 für die Dichtlippenpaarung 9, 18 der Hilfskammer 15. Als Grenze zwischen den Dichtstreifen 11, 16 kann auf Ober- und Unterseite (gesehen in Achsrichtung des Rings) des Doppeldichtrings 20 eine umlaufende Rinne 22, 23 vorgesehen werden. Die Hilfskammer 15 steht mit der von der Testkammer 2 abgewandten Rinne 23 in Verbindung. Die beiden Rinnen 22, 23 werden bevorzugt an wenigstens einer Stelle durch ein (axiales) Loch 24 miteinander verbunden. Rinne und Loch werden so dimensioniert, daß sie als Durchgangsweg des Sterilisationsmittels geeignet sind.

Der Doppeldichtring 20 kann grundsätzlich jede beliebige Form, im wesentlichen in einer Ebene 21, besitzen. Technisch am einfachsten werden die Herstellung und die Handhabung, wenn für die Einzelteile - soweit zutreffend - die Kreissymmetrie, insbesondere mit konzentrischen Dichtstreifen 11 und 16, vorgesehen wird. Ein Vorteil der Ausgestaltung nach Fig. 2 bis 4 gegenüber Fig. 1 besteht darin, daß der Deckel 8 (Kammerkappe) und der Hilfsdeckel 12 (Überkappe) aus einem einzigen Bauteil bestehen.

Fig. 2 zeigt neben der speziellen Ausgestaltung von Hilfskammer 15, Doppeldichtring 20 usw. noch zwei im Rahmen der Erfindung bevorzugte Ausgestaltungen, für den Fall, daß das Zuleitungsmittel der Testkammer 2 als Schlauch 5 ausgebildet ist. Dieser soll in Fig. 2 bei Betrieb unverrückbar und zur Platzersparnis, insbesondere als Wendel 25, um den Behälter 26 der Testkammer 2 gewickelt werden. Zusätzlich kann es vorteilhaft sein, über den Schlauch 5, bzw. die Wendel 25, und den Anschluß 4 eine Schutzkappe 27 zu stülpen, die wenigstens eine Öffnung 4A als Einlaß für das zu registrierende Sterilisationsmittel 6 besitzt. Durch eine solche Schutzkappe 27 wird erreicht, daß die ganze Testvorrichtung 1 ein relativ kleines Außenvolumen behält und der Anschluß 4 bei Anwendung praktisch keinen mechanischen Belastungen mehr ausgesetzt werden kann. Zusätzlich oder alternativ der Auslaß 4 auch vorteilhaft schützen, wenn er, bevorzugt auf der Behälterperipherie 28 der Testkammer 2, von der Wicklung des Zuleitungsmittels 5 bzw. des Schlauchs (abweichend von Fig. 2) überdeckt wird.

Eine weitere bevorzugte Ausgestaltung wird in Fig. 2 mit einem in die Testkammer 2 eingebrachten Pfropfen 29 aus hydrophilen Material dargestellt. Das Material wird nach Fig. 2 in Form des Pfropfens 29 zwischen dem Anschluß 4 der Testkammer 2 und dem in der Testkammer 2 befindlichen Hauptindikator 3 so positioniert, daß in Pfeilrichtung in die Testkammer 2 eindringendes Sterilisationsmittel 6 durch den Pfropfen 29 fließen muß. Noch besser wird die Wirkung des Pfropfens 29, wenn dieses zum Entfeuchten vorgesehene Bauteil nicht erst in der Testkammer 2 (in der Nähe des Hauptindikators 3), sondern bereits am Einlaß 30 des Zuleitungsmittels 5 vorgesehen wird. Ein entsprechendes Ausführungsbeispiel wird in Fig. 1 schematisch dargestellt.

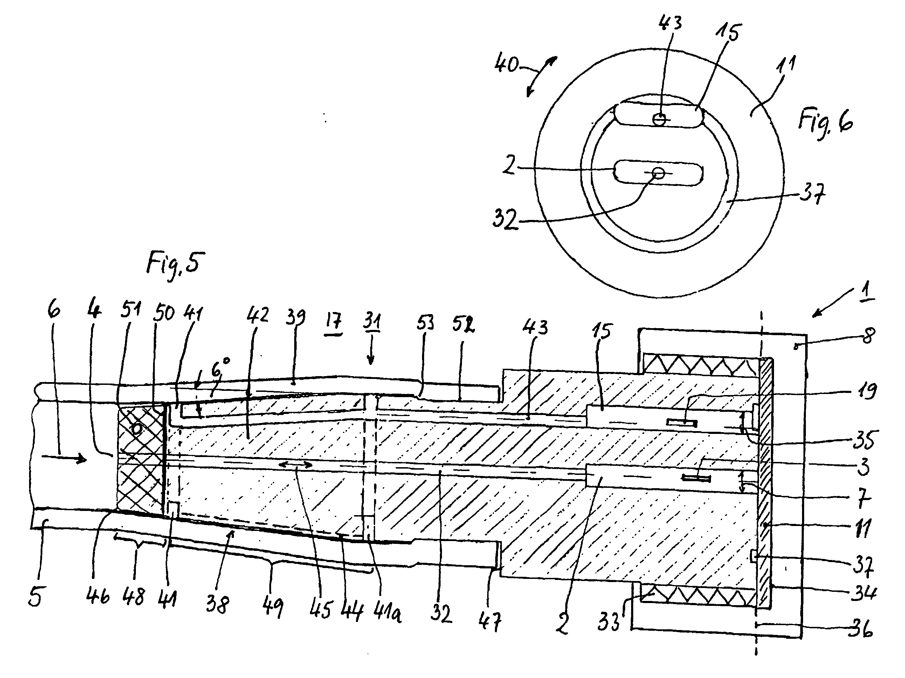

Ein im Rahmen der Erfindung bevorzugtes Ausführungsbeispiel wird anhand von Fig. 5 und 6 erläutert. Die Sterilisationstestvorrichtung 1 nach Fig. 5 umfaßt einen Prüfkörper 31, in den die zur Aufnahme des Hauptindikators vorgesehene Testkammer 2 und zugleich die zur Aufnahme des Hilfsindikators 19 vorgesehene Hilfskammer 15 integriert sind. Innerhalb des Prüfkörpers 31 führt eine Hauptleitung 32 von der Anschlußstelle 4 zur Testkammer 2. Die Kontrollöffnung 7 der Testkammer 2 wird mit einem Deckel 8, insbesondere Schraubdeckel mit Gewinde 33, verschlossen, der auf der Deckelinnenfläche 34 eine einzige Dichtung 11, bevorzugt als kreisförmige Dichtplatte, besitzt.

Räumlich neben der Testkammer 2 wird im Prüfkörper 31 die Hilfskammer 15 so angeordnet, daß ihre Kontrollöffnung 35 ebenfalls mit Hilfe der einen Dichtung 11 zu verschließen ist. Die beiden Kontrollöffnungen 7 und 35 befinden sich im Ausführungsbeispiel nach Fig. 5 bzw. 6 in ein und derselben Abschlußebene 36 des Prüfkörpers 31. In dieser Abschlußebene 36 wird im Ausführungsbeispiel ein sich um die Testkammer 2 bzw. deren Kontrollöffnung 7 mit Abstand herum erstreckender Ringkanal 37 vorgesehen, der in die Hilfskammer 15 bzw. deren Kontrollöffnung 35 mündet (die Kontrollöffnung 35 kreuzt). Wenn also die Dichtung 11 an der Deckelinnenfläche 34 undicht sein sollte und daher aus dem die Sterilisationstestvorrichtung 1 umgebenden Außenraum 17 Sterilisationsmittel durch das Gewinde 33 und längs der Dichtung 11 in Richtung Testkammer 2 vordringen sollte, muß dieser Leckstrom vor der Testkammer 2 den Ringkanal 37 passieren. Über den Ringkanal 37 dringt aber das Sterilisationsmittel in die Hilfskammer 15 ein und verändert den dort befindlichen Hilfsindikator 19.

Im Prinzip ähnlich wie an den Kontrollöffnung 7 bzw. 35 wird die Dichtung der erfindungsgemäßen Vorrichtung nach Fig. 5 an der Kupplung des Zuleitungsmittels 5 kontrolliert. In dem gezeichneten Ausführungsbeispiel wird angenommen, daß ein als Schlauch ausgebildetes Zuleitungsmittel 5 vorgesehen ist und daß der Prüfkörper 31 einen mit einem Längsdurchgang, der Hauptleitung 32, zur Testkammer 2 ausgestatteten Stutzen 38 zum Aufschieben eines als Dichtung wirkenden Schlauchendes 39 besitzt. Ferner wird angenommen, daß im Stutzenumfang mindestens ein - nach dem Aufschieben des als Schlauch ausgebildeten Zuleitungsmittels 5 - von diesem eingeschlossenen, in Richtung des Stutzenumfangs 40 geschlossener und mit der Hilfskammer 15 verbundener Ableitkanal 41 vorgesehen ist. Vorzugsweise soll ein Stutzen 38 mit einem sich in das jeweilige Zuleitungsmittel 5 hinein erstreckenden Konusteil 42 vorgesehen werden und der Ableitkanal 41 soll im Bereich dieses Konusteils 42 am Stutzenumfang vorgesehen sein. Der Ableitkanal 41 wird in der Regel als Ringkanal, also als sich in Stutzenumfangsrichtung erstrekkender Kanal ausgebildet. Er soll eine sich zumindest zum Teil durch das Innere des Prüfkörpers 31 erstreckende Verbindungsleitung 43 zur Hilfskammer 15 besitzen. In den Ableitkanal 41 eingedrungenes Sterilisationsmittel gelangt dann also auch an den Hilfsindikator 19.

Wenn innerhalb eines Konusteils 42, dessen Durchmesser in der Größenordnung von 1 cm liegt, nicht genug Platz für zwei Leitungen (die Hauptleitung 32 und die Verbindungsleitung 43) sein sollte, kann es günstig sein, die Verbindungsleitung im betreffend den Durchmesser dickeren Teil des Prüfkörpers 31 innerhalb dieses Körpers und im Bereich des Konusteils 42 in einer gestrichelt angedeuteten Rinne 44 auf der Konusfläche (in Richtung der Konusneigung) entlang zu führen. In diesem Fall kann es günstig sein, am Übergang zwischen Rinne 44 und Verbindungsleitung 43 einen weiteren Ableitkanal 41a um den Konus herum vorzusehen.

In einem Ausführungsbeispiel hatte der gesamte Prüfkörper 31 nach Fig. 5 eine Länge von 56 mm, der Konusteil 42 war 21 mm lang und hatte eine Steigung von etwa 6°. Sein Durchmesser lag zwischen 13 und 9 mm. Die lichte Weite der Hauptleitung 32 und der Verbindungsleitung 33 betrug etwa 1 mm. Die Breite des Ableitkanals 41 betrug in Längsrichtung 45 des Prüfkörpers etwa 1 mm. Die Tiefe betrug etwa 2 mm. Die beiden Kammern 2 und 15 waren etwa gleich groß, sie hatten eine Länge von 15 mm und einen Durchmesser von 2 mm. Der Ableitkanal 37 in der Abschlußebene 36 des Prüfkörpers 31 hatte eine Kanaltiefe von 0,5 mm, eine Kanalbreite von 2 mm und einen Innenradius von 12 mm.

Wenn das Zuleitungsmittel 5 als Schlauch ausgebildet wird, ist es für eine praktikable Handhabung günstig, wenn der Innendurchmesser des Schlauchs geringfügig, z.B. (bei den vorgenannten Maßen) 1 mm größer als der Durchmesser des freien Endes 46 des Konusteils 42 gemacht wird. Nach dem Aufschieben des Schlauchs, beispielsweise bis zu dem in Fig. 5 dargestellten Anschlag 47, wird dann die Dichtwirkung des Schlauchs in einer an das freie Ende 47 angrenzenden ersten Konuszone 48 ungenügend. Erst mit zunehmendem Konusdurchmesser in Richtung Anschlag 47 wird eine vollkommene Dichtung zwischen Schlauch und Konus erreicht. Dieser Konusbereich mit (bei unversehrtem Zuleitungsmittel 5) vollkommener Dichtung wird als zweite Konuszone 49 bezeichnet. Die Grenze 50 zwischen erster und zweiter Konuszone 48/49 läßt sich experimentell bestimmen, z.B. indem man auf der Konusoberfläche die dargestellte Rinne 44 stückweise vom Hilfs-Ringkanal 41a in Richtung freies Konusende 46 verlängert. Wenn die Rinne in der zweiten Konuszone bleibt, kann (bei unversehrter Zuleitung) kein Sterilisationsmittel bis zur Hilfskammer 15 vordringen. Wenn aber die Rinne in die erste Zone hineinreicht, kann das Sterilisationsmittel durch die Rinne 44 bis zur Hilfskammer 15 gelangen und den Hilfsindikator 19 verändern. Wenn auf diese Weise die Position der Grenze 50 bestimmt ist, wird nach einer bevorzugten Ausgestaltung der Erfindung der Ableitkanal 41 so nahe an die Grenze 50 gelegt, daß bei unversehrter Zuleitung 5 gerade kein Sterilisationsmittel in den Ableitkanal 41 einströmen kann.

Wenn bei der vorbeschriebenen Position des Ableitkanals 41 in der Nähe, der Abstand kann in der Größenordnung von 1 mm sein, der Grenze 50 liegt, und eine Beschädigung des über den Konus gestülpten Schlauchs vorliegt,. sind zwei Fälle zu unterscheiden:

Wenn die Beschädigung am Prüfkörper bzw. Konus oberhalb des Ableitkanals 41 (im dickeren Konusbereich) liegt, kann das Sterilisationsmittel, sofern es durch die Zuleitung dringt, nur über den Ableitkanal 41 und die Verbindungsleitung 43 (gegebenenfalls die Rinne 44) in die Hilfskammer 15 fließen. Wenn dagegen die Undichtheit, z.B. ein Loch 51 in der ersten Zone 48 auftritt, kann durch dieses Loch von außen Sterilisationsmittel in dem Bereich zwischen Schlauch und der ersten Zone 48 am freien Ende 46 des Konusteils 42 eindringen. Der Weg vom Loch 51 zur Grenze 50 ist dann auf jeden Fall kürzer als der Weg vom freien Ende 46 zur Grenze 50. Das bedeutet, daß durch das Loch einströmendes Sterilisationsmittel zumindest auch zum Ableitkanal 41 gelangen muß. Durch die Erfindung wird also erreicht, daß eine als Schlauch ausgebildete Zuleitung 5, die zum Aufstecken auf den Prüfkörper aufgeweitet werden muß und dabei verletzt werden kann, als unbrauchbar ausgeschieden wird, weil jedes in der ersten Zone 48 oder in der zweiten Zone 49 auftretende Loch des Schlauchs zu einer Veränderung des Hilfsindikators 19 führt.

Nebenbei sei darauf hingewiesen, daß es für einen festen Sitz einer als Schlauch ausgebildeten Zuleitung 5 auf dem Prüfkörper 31 günstig ist, wenn der Bereich 52 in der Nähe des Anschlags 47 mit geringerem Durchmesser, insbesondere im Anschluß an einen Absatz 53, ausgebildet wird. Es ist nämlich zu berücksichtigen, daß zumindest zu Beginn der Messung der Innendruck der erfindungsgemäßen Vorrichtung größer als der Außendruck ist. Außerdem kann es wie oben schon erläutert, günstig sein, in das freie Ende der Zuleitung 5 einen Pfropfen 29 aus hydrophilem bzw. hygroskopischem Material einzusetzen.

### Bezugszeichenliste

- 1 =: Sterilisationstestvorrichtung
- 2 =: Testkammer
- 3 =: Hauptindikator
- 4 =: Anschluß (2)
- 5 =: Zuleitungsmittel
- 6 =: Sterilisationsmittel
- 7 =: Kontrollöffnung (2)
- 8 =: Deckel
- 9 =: Dichtlippe (2)
- 10 =: Dichtlippe (18)
- 11 =: Kammerdichtung
- 12 =: Hilfsdeckel
- 13 =: Gewinde (18)
- 14 =: Gewinde (12)
- 15 =: Hilfskammer
- 16 =: Hilfsdichtung
- 17 =: Außenraum
- 18 =: Dichtlippe (12)
- 19 =: Hilfsindikator
- 20 =: Doppeldichtring
- 21 =: Ebene
- 22, 23 =: Rinne
- 24 =: Loch
- 25 =: Wendel
- 26 =: Behälter
- 27 =: Schutzkappe
- 28 =: Peripherie (2)
- 29 =: Pfropfen
- 30 =: Einlaß (5)
- 31 =: Prüfkörper
- 32 =: Hauptleitung
- 33 =: Gewinde
- 34 =: Deckelinnenfläche
- 35 =: Kontrollöffnung (15)
- 36 =: Abschlußebene
- 37 =: Ringkanal
- 38 =: Stutzen
- 39 =: Schlauchende
- 40 =: Umfangsrichtung
- 41 =: Ableitkanal
- 42 =: Konusteil
- 43 =: Verbindungsleitung
- 44 =: Rinne
- 45 =: Längsrichtung (31)
- 46 =: freies Ende (42)
- 47 =: Anschlag
- 48 =: erste Konuszone
- 49 =: zweite Konuszone
- 50 =: Grenze (48/49)
- 51 =: Loch
- 52 =: enger Bereich
- 53 =: Absatz (52)

## Patentansprüche

1. Sterilisationstestvorrichtung (1) mit einer in einen Prüfkörper (31) integrierten und zur Aufnahme eines Indikators (3) vorgesehenen Testkammer (2), die vorgegebene Zuleitungsmittel (5) für das jeweilige Sterilisationsmittel (6) sowie eine mit einem Kammerdeckel (8) zu verschließende Kontrollöffnung (7) besitzt und außer am Einlaß (30) des Zuleitungsmittels (5) mit einer Testkammerdichtung (11, 39) gegen ein Eindringen des Sterilisationsmittels (6) ausgestattet ist,
**dadurch gekennzeichnet,**
**daß** in der Testkammerdichtung (11, 39) im Wege eines gegebenenfalls die Zuleitungmittel (5) umgehenden Lecks (51) mindestens ein an eine zur Aufnahme eines Hilfsindikators (19) vorgesehene Hilfskammer (15) angeschlossener Ableitkanal (37, 41) vorgesehen ist, wobei die Hilfskammer (15) bei einwandfreier Testkammerdichtung (11; 39) luftdicht verschlossen ist.

2. Sterilisationstestvorrichtung mit einer in einen Prüfkörper (31) integrierten und zur Aufnahme eines Indikators (3) vorgesehenen Testkammer (2), die vorgegebene Zuleitungsmittel (5) für das jeweilige Sterilisationsmittel (6) sowie eine mit einem Kammerdeckel (8) zu verschließende Kontrollöffnung (7) besitzt und außer am Einlaß (30) des Zuleitungsmittels (5) mit einer Testkammerdichtung (11, 39) gegen ein Eindringen des Sterilisationsmittels (6) ausgestattet ist,
**dadurch gekennzeichnet,**
**daß** in der Testkammerdichtung (11, 39) im Wege eines gegebenenfalls die Zuleitungsmittel (5) umgehenden Lecks (51) mindestens eine an eine zur Aufnahme eines Hilfsindikators (19) vorgesehene, bei einwandfreier Testkammerdichtung (11, 39) luftdicht gekapselte Hilfskammer (15) angeschlossener Ableitkanal (37, 41) vorgesehen ist und daß ein als Schlauch ausgebildetes Zuleitungsmittel (5) vorgesehen ist, wobei der Prüfkörper (31) einen mit einem Längsdurchgang (32) zur Testkammer (2) ausgestatteten Stutzen (38) zum Aufschieben eines dichtenden Schlauchendes (39) besitzt und wobei im Stutzenumfang mindestens ein - nach dem Aufschieben des Schlauchs von diesem eingeschlossenen - in Richtung (40) des Stutzenumfangs geschlossener und mit einer Hilfskammer (15) verbundener Ableitkanal (41) vorgesehen ist.

3. Vorrichtung nach Anspruch 1 oder 2,
**dadurch gekennzeichnet,**
**daß** eine Hilfskammer (15) mit eigener Kontrollöffnung (35) im Prüfkörper (31) räumlich neben der Testkammer (2) angeordnet ist, daß ein die Testkammer-Kontrollöffnung (7) umgebender Ableitkanal (37) vorgesehen ist und daß die beiden, vorzugsweise in einer Ebene (36) liegenden, Kontrollöffnungen (7, 35) mit ein und derselben Dichtung (11) eines einzigen Kammerdeckels (8) verschließbar sind.

4. Vorrichtung nach Anspruch 2 oder 3,
**dadurch gekennzeichnet,**
**daß** ein Stutzen (38) mit einem sich in den jeweiligen Schlauch hinein erstreckenden Konusteil (42) vorgesehen ist und der Ableitkanal (41) im Bereich des Konusteils (42) vorgesehen ist.

5. Vorrichtung nach mindestens einem der Ansprüche 2 bis 4,
**dadurch gekennzeichnet,**
**daß** eine zumindest zum Teil innerhalb des Prüfkörpers (31) verlaufende Verbindung (43) zwischen Ableitkanal (41) und Hilfskammer (15) vorgesehen ist.

6. Vorrichtung nach mindestens einem der Ansprüche 2 bis 5,
**dadurch gekennzeichnet,**
**daß** die Verbindung zwischen Ableitkanal (41) und Hilfskammer (15) zumindest zum Teil als Rinne (44) an der Oberfläche des Konusbereichs ausgebildet ist.

7. Vorrichtung nach Anspruch 6,
**dadurch gekennzeichnet,**
**daß** am Übergang von Rinne (44) und innerhalb des Prüfkörpers (31) verlaufender Verbindung (43) ein sich um den Prüfkörper (31), insbesondere in dessen Konusbereich, herum erstreckender Ableitkanal (41a) vorgesehen ist.

8. Vorrichtung nach mindestens einem der Ansprüche 1 bis 7,
**dadurch gekennzeichnet,**
**daß** in das vom Prüfkörper (31) abgewandte freie Ende bzw. den Einlaß (30) des Zuleitungsmittels (5), insbesondere Schlauchs, ein Pfropfen (29) aus hydrophilem Material eingesetzt ist.

9. Verfahren zum Betrieb der Vorrichtung nach mindestens einem der Ansprüche 1 bis 8,
**dadurch gekennzeichnet,**
**daß** zwischen die Testkammer (2) und ein Leck (51) der Testkammerdichtung (39) eine Hilfskammer (15) mit Hilfsindikator (19) geschaltet wird.

## Claims

1. Sterilisation test device (1) with a test chamber (2) integrated in a test piece (31) and provided for receiving an indicator (3), the chamber (2) comprising predetermined feed means (5) for the respective sterilising agent (6) and an inspection opening (7) to be closed by a chamber cover (8) and, apart from the inlet (30) of the feed means (5), being equipped with a test chamber seal (11, 39) against penetration of the sterilising agent (6), **characterised in that** at least one discharge duct (37, 41) connected to an auxiliary chamber (15) provided for receiving an auxiliary indicator (19) is provided in the test chamber seal (11, 39) in the path of a leak (51) possibly bypassing the feed means (5), the auxiliary chamber (15) being sealed in an air-tight manner when the test chamber seal (11; 39) is intact.

2. Sterilisation test device (1) with a test chamber (2) integrated in a test piece (31) and provided for receiving an indicator (3), the chamber (2) comprising predetermined feed means (5) for the respective sterilising agent (6) and an inspection opening (7) to be closed by a chamber cover (8) and, apart from the inlet (30) of the feed means (5), being equipped with a test chamber seal (11, 39) against penetration of the sterilising agent (6), **characterised in that** at least one discharge duct (37, 41) connected to an auxiliary chamber (15) encapsulated in an air-tight manner when the test chamber seal (11, 39) is intact and provided for receiving an auxiliary indicator (19) is provided in the path of a leak (51) possibly bypassing the feed means (5), and **in that** a feed means (5) designed as a tube is provided, the test piece (31) comprising a connection piece (38) for sliding on a sealing tube end (39) and equipped with a longitudinal passage (32) to the test chamber (2), and at least one discharge duct (41) closed in the direction (40) of the circumference of the connection piece and connected to an auxiliary chamber (15) and enclosed by the tube after it has been slipped on, being provided in the circumference of the connection piece.

3. Device according to claim 1 or 2, **characterised in that** an auxiliary chamber (15) with its own inspection opening (35) is arranged spatially close to the test chamber (2) in the test piece (31), **in that** a discharge duct (37) surrounding the test chamber inspection opening (7) is provided and **in that** the two inspection openings (7, 35) preferably located in one plane (36) can be sealed by one and the same seal (11) of a single chamber cover (8).

4. Device according to claim 2 or 3, **characterised in that** a connection piece (38) with a conical portion (42) extending into the respective tube is provided and the discharge duct (41) is provided in the region of the conical portion (42).

5. Device according to at least any one of claims 2 to 4, **characterised in that** a connection (43) extending at least partially inside the test piece (31) is provided between discharge duct (41) and auxiliary chamber (15).

6. Device according to at least any one of claims 2 to 5, **characterised in that** the connection between discharge duct (41) and auxiliary chamber (15) is at least partially formed as a groove (44) on the surface of the conical region.

7. Device according to claim 6, **characterised in that** a discharge duct (41a) extending round the test piece (31), in particular in the conical region thereof, is provided at the transition from groove (44) and connection (43) extending inside the test piece (31).

8. Device according to at least any one of claims 1 to 7, **characterised in that** a stopper (29) made of hydrophilic material is inserted into the free end remote from the test piece (31) or the inlet (30) of the feed means (5), in particular the tube.

9. Method for operating the device according to at least any one of claims 1 to 8, **characterised in that** an auxiliary chamber (15) with auxiliary indicator (19) is connected between the test chamber (2) and a leak (51) of the test chamber seal (39).

## Revendications

1. Dispositif de test (1) pour stérilisation incluant une chambre de test (2) qui est intégrée dans une éprouvette (31) et prévue pour recevoir un indicateur (3), possède un moyen de conduite d'amenée (5) pour le milieu de stérilisation respectif (6) et une ouverture de contrôle (7) à fermer par un couvercle (8) de chambre et est équipée à l'extérieur, à l'entrée (30) du moyen de conduite d'amenée (5), d'un joint étanche (11, 39) de chambre de test pour empêcher une pénétration du milieu de stérilisation (6),
**caractérisé en ce que**
au moins un canal de dérivation (37, 41) raccordé à une chambre auxiliaire (15) prévue pour recevoir un indicateur auxiliaire (19) est prévu dans le joint étanche (11, 39) de chambre de test dans le trajet d'une fuite (51) qui contournerait éventuellement le moyen de conduite d'amenée (5), la chambre auxiliaire (15) étant fermée de façon étanche lorsque le joint (11; 39) de la chambre de test est irréprochable.

2. Dispositif de test pour stérilisation incluant une chambre de test (2) qui est intégrée dans une éprouvette (31) et prévue pour recevoir un indicateur (3), possède un moyen de conduite d'amenée (5) pour le milieu de stérilisation respectif (6) et une ouverture de contrôle (7) à fermer par un couvercle (8) de chambre et est équipée à l'extérieur, à l'entrée (30) du moyen de conduite d'amenée (5), d'un joint étanche (11, 39) de chambre de test pour empêcher une pénétration du milieu de stérilisation (6),
**caractérisé en ce que**
au moins un canal de dérivation (37, 41) raccordé à une chambre auxiliaire (15) enrobée de façon étanche à l'air prévue pour recevoir un indicateur auxiliaire (19) est prévu dans le joint étanche (11, 39) de chambre de test dans le trajet d'une fuite (51) qui contournerait éventuellement le moyen de conduite d'amenée (5), et **en ce que** le dispositif comprend un moyen de conduite d'amenée (5) consistant en un tube souple, l'éprouvette (31) possède un piquage (38) équipé d'un passage longitudinal (32) vers la chambre de test (2) pour y enfiler le tube souple et au moins un canal de dérivation (41) fermé en direction (40) de la périphérie du piquage et connecté à une chambre auxiliaire (15) est prévu dans la périphérie du piquage - et est fermé par le tube souple lorsque celui-ci a été enfilé.

3. Dispositif selon la revendication 1 ou 2,
**caractérisé en ce que**
une chambre auxiliaire (15) à ouverture propre de contrôle (35) est agencée près de la chambre de test (2) dans l'espace de l'éprouvette (31), **en ce qu'**un canal de dérivation (37) qui entoure l'ouverture de contrôle (7) de la chambre de test est prévue, et **en ce que** les deux ouvertures de contrôle (7, 35) situées de préférence dans un plan (36) peuvent être fermées au moyen d'un seul joint étanche (11) d'un couvercle unique (8) de chambre.

4. Dispositif selon la revendication 2 ou 3,
**caractérisé en ce que**
un piquage (38) à élément conique (42) s'étendant vers l'intérieur dans le tube souple respectif est prévu et **en ce que** le canal de dérivation (41) est prévu dans la zone de l'élément conique (42).

5. Dispositif selon l'une quelconque des revendications 2 à 4,
**caractérisé en ce que**
une connexion (43) dont le trajet est au moins en partie à l'intérieur de l'éprouvette (31) est prévue entre le canal de dérivation (41) et la chambre auxiliaire (15).

6. Dispositif selon l'une quelconque des revendications 2 à 5,
**caractérisé en ce que**
la connexion entre le canal de dérivation (41) et la chambre auxiliaire (15) consiste au moins en partie en une rainure (44) ménagée dans la surface de la zone conique.

7. Dispositif selon la revendication 6,
**caractérisé en ce que**
un canal de dérivation (41a) qui s'étend autour de l'éprouvette (31), en particulier dans sa zone de cône, est prévue au raccordement entre la rainure (44) et la connexion (43) de tracé interne à l'éprouvette (31).

8. Dispositif selon l'une quelconque des revendications précédentes,
**caractérisé en ce que**
un bouchon (29) en matière hydrophile est introduit dans l'extrémité libre opposée à l'éprouvette (31) ou l'entrée (30) du moyen de conduite d'amenée (5), en particulier du tube souple.

9. Procédé de mise en oeuvre du dispositif selon l'une quelconque des revendications précédentes,
**caractérisé en ce que**
une chambre auxiliaire (15) à indicateur auxiliaire (19) est montée entre la chambre de test (2) et une fuite (51) du joint étanche (39) de chambre de test.
